(19) Europäisches Patentamt

European Patent Office

Office européen des brevets

(11) **EP 1 190 722 B1**

(12) **EUROPÄISCHE PATENTSCHRIFT**

(45) Veröffentlichungstag und Bekanntmachung des Hinweises auf die Patenterteilung:
**19.04.2006   Patentblatt 2006/16**

(51) Int Cl.:
*A61L 15/26* (2006.01)    *A61L 15/58* (2006.01)

(21) Anmeldenummer: **01120367.6**

(22) Anmeldetag: **25.08.2001**

(54) **Verband**

Dressing

Pansement

(84) Benannte Vertragsstaaten:
**AT BE CH CY DE DK ES FI FR GB GR IE IT LI LU MC NL PT SE TR**

(30) Priorität: **22.09.2000   DE 10047673**

(43) Veröffentlichungstag der Anmeldung:
**27.03.2002   Patentblatt 2002/13**

(73) Patentinhaber: **Beiersdorf Aktiengesellschaft**
**20245 Hamburg (DE)**

(72) Erfinder:
- **Ahrens, Helge**
  **22297 Hamburg (DE)**
- **Hartkopf, Carsten**
  **22303 Hamburg (DE)**
- **Kenndoff, Jochen, Dr.**
  **65116 Malang,**
  **Jawa Timur (ID)**
- **Köhler, Ulrich, Dr.**
  **22607 Hamburg (DE)**
- **Lenz, Dirk, Dr.**
  **Beford, MK43 0AL (GB)**
- **Mayan, Robert, Dr.**
  **21614 Buxtehude (DE)**
- **Sachau, Günther**
  **25451 Quickborn (DE)**
- **Wesselkamp, Ingrid**
  **22145 Hamburg (DE)**

(56) Entgegenhaltungen:
EP-A- 0 147 588          EP-A- 0 264 299
EP-A- 0 919 211          EP-A- 1 190 723
WO-A-92/05755

EP 1 190 722 B1

**Beschreibung**

Die Erfindung betrifft einen mehrlagigen Verband, der insbesondere an Druckstellen, an Blasen und offenen Blasen an der Ferse, am Handballen und an den Fingern eingesetzt werden kann.

Druckstellen, Blasen und offene Blasen an der Ferse, am Handballen und an den Fingern werden üblicherweise durch konventionelle Pflasterprodukte versorgt, die aus einem textilen Trägermaterial, einer Haftklebeschicht und einer Vlies-Wundauflage bestehen.

Derartige bewährte Produkte sind beispielsweise von der Firma Beiersdorf erhältlich, zum Beispiel unter dem Namen Hansäplast ® elastic oder Hansaplast ® classic.

Derartige Pflaster weisen allerdings bei der Verwendung einige Nachteile auf:

- Durch Reibung im Schuh kommt es zum allmählichen Ablösen des Pflasters
- Verschmutzung des Trägermaterials bei längerer Tragedauer
- Geringe Polsterwirkung
- Druckstellen am Rand des Pflasters
- Schmerzhaft beim Entfemen

Moderne Wundversorgungsprodukte wie Hydrokolloide (siehe hierzu zum Beispiel. "Hydrokolloide" von R. Lipmann in "Medical Device & Diagnostic Industry, June 1999), die für Kolostomie- und professionelle Wundversorgungsanwendungen entwickelt wurden, finden zunehmend Anwendung für Druckstellen und Blasen.

Wundversorgungsprodukte auf Basis von Hydrokolloiden weisen Vorteile gegenüber herkömmlichen Pflastern auf. Diese generieren ein feuchtes Wundheilungsmilieu, das die Wunde nicht austrocknen läßt und ein optimales Milieu zur schnellen Wundheilung erzeugt. Weitere Vorteile sind die Unauffälligkeit bei der Anwendung, sicheres Haftvermögen, Absorptionsvermögen von Exudat (zum Beispiel aus einer Blase stammend), gute Polsterwirkung und die schmerzlose Entfernbarkeit.

Typische Zusammensetzungen der ersten kommerziell erhältlichen Hydrokolloide für Wundversorgungsprodukte umfassen:

- Niedermolulares Polyisobutylen (40 Gew.-%),
- Pektin (20 Gew.-%),
- Natriumcarboxymethylcellulose = CMC (20 Gew.-%)
- Gelatine (20 Gew.-%)

Moderne Hydrokolloid-Formulierungen, sogenannte integrierte Formulierungen, wie sie zum Beispiel von der Firma Coloplast angeboten werden, basieren auf Styrol-Isopren-Styrol-Blockpolymeren mit Kohlenwasserstoffharzen ("hydrocarbon resins") als Tackifiem, Mineralöl als Weichmacher sowie CMC als Absorber.

Diese Formulierungen mit SIS als Gerüstbildner enthalten glasartige Domänen (Styrol-Blöcke) und thermoplastische Domänen (Isopren-Blöcke). Bei Raumtemperatur sorgen die glasartigen Domänen für eine Art dreidimensionale "cross-linking"-Struktur, die bei höherer Temperatur aufgehoben wird.

Hydrokolloide zeigen auch bei feuchten Bedingungen einen guten "wet-tack", so daß sie für die Anwendung als Blasenpflaster an Ferse und Handinnenfläche, einem Applikationsort mit relativ hohem Feuchtigkeitsverlust der Haut, gut geeignet sind.

Aus der EP 0 264 299 B1 ist ein Verband bekannt, der aus einem wasserabsorbierenden Abdichtungskissen besteht, das seinerseits von einem oder mehreren Hydrokolloiden gebildet wird. Das oder die Hydrokolloide sind in einem Bindemittel aufgelöst oder mit demselben gemischt.

Das Kissen ist fest von einer wasserdichten Deckungsschicht vollständig umfaßt. Erfindungsgemäß ist das Kissen mindestens um die äußere Peripherie auf eine solche Weise abgeschrägt, daß die Dicke am Rand etwa ein Viertel ihrer maximalen Dicke nicht über schreitet.

Hydrogele sind makromolekulare, natürliche oder synthetische Stoffe, die aufgrund eines hohen Gehaltes an hydrophilen Gruppen in der Lage sind, Wasser absorbtiv zu binden.

Die Wasseraufnahmekapazität vieler Hydrogele beträgt das Mehrfache des Eigengewichtes der wasserfreien Substanz.

Hydrogele werden in vielfältiger Form in der Wundversorgung eingesetzt, denn sie schützen Wunden vor der Austrocknung, saugen Wundsekret auf, dienen als Matrix für Wirkstoffe aller Art und auch als Basis für die Besiedelung mit autologen oder heterologen Hautzellen.

Hydrogele können unter anderem in Form von Schäumen verwendet werden. Schäume zur Versorgung von Hautwunden oder chirurgischen Wunden sind dem Fachmann an sich bekannt. Hauptsächlich finden dabei Polyurethanschäume oder Kollagenschäume Verwendung.

Auch selbstklebende Gelschäume sind dem Fachmann bekannt Diese lassen sich zwar im allgemeinen recht gut auf der Haut fixieren, haben aber meistens den Nachteil, daß ihre Wasseraufnahmekapazität und Wasserrückhaltevermögen stark eingeschränkt sind.

Weiterhin sind hydrophile Schäume aus Polyurethangelen bekannt. Die WO 88/01878 A1 beschreibt selbstklebende Polyurethanschäume bzw. Polyurethanschaumgele, welche unter anderem einpolymerisierte Methacrylate enthalten können. Die Herstellung dieser Schaumgele erfolgt durch Zusatz von Wasser.

Polyurethangele auf der Basis einer Polyurethanmatrix und höhermolekularen Polyolen werden auch in EP 0 057 839 B1 beschrieben. Selbsthaftende Flächengebilde aus Polyurethangelen sind aus EP 0 147 588 B1 bekannt. Die in diesen beiden letztgenannten Schriften offenbarten Polyurethangele sind ungeschäumt.

Die selbstklebenden Gele haben Isocyanatkennzahlen von 15 bis 70 (EP 0147 588 A2).

EP 0 196 364 A2 beschreibt hydrophile Polyurethanschäume, die mit wasserabsorbierenden Polymeren auf Basis eines Copolymers der Acrylsäure und Kaliumacetat gefüllt sein können und für medizinische Zwecke gedacht sind. Das Polyurethan wird auf Basis von MDI hergestellt. Der eingesetzte Polyether hat eine Mindestfunktionalität von zwei Hydroxylgruppen, bevorzugt je zwei bis drei Hydroxylgruppen. Das Verhältnis NCO/OH ist stöchiometrisch. Damit handelt es sich nicht um ein gelförmiges Polyurethan. Geschäumt kann mit Druckluft oder mit anderen, nicht mit dem Isocyanat reagierenden Gasen oder mit Hilfe von niedrig siedenden Lösungsmitteln werden. Die Mischung von Absorber mit Polyetherpolyol erfolgt etwa im Verhältnis 3:1. Der Schaum hat klebende Eigenschaften auf Wunden, die durch aluminisiertes Vlies gänzlich unterdrückt werden müssen, um ihn zur Wundbehandlung einsetzen zu können.

Schaumwundauflagen, wie sie zum Beispiel von der Firma Beiersdorf unter dem Namen Cutinova® thin und Cutinova® hydro erhältlich sind, sind u. a. beschrieben in DE 42 33 289A1, in DE 196 18 825 A1 und WO 97/43328.

Danach besteht der Polyurethangelschaum aus einem Polyadditionsprodukt eines Polyetherpolyols (Levagel ® von Bayer AG) mit einem aromatischen oder aliphatischen Diisocyanat (Desmodur ® Bayer AG), in das ein Polyacrylat-Superabsorber-Pulver (Favor ®, Stockhausen) eingearbeitet wurde. Das Polyurethangel kann, je nach Verhältnis von OH-Äquivalenten des Polyols zu reaktiven Isocyanat-Gruppen, schwach oder stark selbsthaftend auf Haut eingestellt werden.

Der flächige Polyurethangelschaum mit einer Dicke von 1 bis 6 mm wird einseitig von einer Polyurethanfolie abgedeckt. Pflaster entsprechender Größe werden aus der Ballenware ausgestanzt. Die so hergestellte Wundauflage entklebt überraschenderweise vollständig bei Aufnahme von Wundflüssigkeit und zeigt dabei nicht die von Hydrokolloiden bekannte Neigung zur Desintegration bei starker Quellung, die dazu führen kann, daß Rückstände des Hydrokolloids in der Wunde verbleiben.

Die ausgestanzten großflächigen Wundauflagen eignen sich hervorragend zur Versorgung von chronischen oder schwerheilenden Wunden von Patienten, die stationär versorgt werden müssen.

Bei kleineren Bagatell-Verfetzungen oder Blasenbildung aufgrund von Druckstellen an Hand, Ballen und Ferse zeigt dieser Produktaufbau jedoch einige Nachteile.

Das Produkt rollt sich aufgrund seiner Stanzränder bei mechanischer Belastung leicht auf. Bei Kontakt mit Feuchtigkeit erweisen sich die offenen Stanzränder als Nachteil, da dadurch Wasser an die saugende Schicht gelangen kann und zur Aufquellung und Entklebung des Polyurethangels führt.

Schließlich können noch herkömmliche Pflaster zur Versorgung von Blasen eingesetzt werden (zum Beispiel das Gewebepflaster Hansaplast ® classic der Firma Beiersdorf), die sich nur bedingt für die Anwendung als Blasenpflaster zur Versorgung von Druckstellen bzw. geschädigter Hornhaut an stark konturierten Körperpartien eignen.

Nachteilig erweisen sich die geringe Elastizität und die Neigung zum Aufrollen des Trägermaterials an den Kanten des Pflasters bei mechanischer Belastung bei längerer Tragedauer. Zusätzlich wird das Pflaster bei täglicher Körperpflege oder beim Händewaschen stark durchfeuchtet und verliert an Haftvermögen.

Herkömmliche Pflaster fallen optisch stark auf, behindern Bewegungen bzw. beeinträchtigen das Tragegefühl im Schuh.

Aufgabe der Erfindung ist es, einen Verband zur Verfügung zu stellen, welcher in der Lage ist, Exudat aus Druckstellen aufzusaugen, welcher gut polstert, welcher ausreichend Feuchtigkeit von der Hornhaut durch das Pflaster nach außen transportiert und welcher ein feuchtes Wundheilungsmilieu erzeugt.

Gelöst wird die Aufgabe durch einen Verband, wie er im Anspruch 1 dargelegt ist. Die Untersprüche umfassen vorteilhafte Varianten des Erfindungsgegenstands.

Demgemäß betrifft die Erfindung einen Verband insbesondere zur Abdeckung von Wunden und Verhütung oder Behandlung von Blasen, umfassend

- eine wasserdampfdurchlässige Trägerfolie, auf die vollflächig
- eine Klebeschicht aufgetragen ist, wobei
- mittig auf der Klebeschicht eine wasserdampfdurchlässige Polyurethan-Matrix vorhanden ist, die sich ausgehend von einem Punkt, insbesondere mittig gelegen, zum Rand der Klebeschicht hin abschrägt, wobei die Peripherie der Klebeschicht zumindest teilweise nicht von der Polyurethan-Matrix bedeckt ist.

Insbesondere liegt der Punkt im Flächenzentrum, um ein symmetrisches Aussehen des Pflasters zu erzielen. Die Abschrägung kann aber auch unregelmäßig erfolgen, je nach Bedarf und Anwendungsfall des Pflasters. Es ergeben sich auf diese Weise unterschiedlichste Formen. Die Matrix kann zum Beispiel linsenförmig geformt oder halbkugelförmig sein.

Die Trägerfolie besteht vorzugsweise aus einer transparenten mehrschichtigen wasserdampfdurchlässigen Polyurethan-Folie, die in einer weiteren vorteilhaften Ausführungsform eine Dicke von 60 bis 80 μm aufweist. Die vorteilhafte Dicke resultiert aus der Anforderung nach einem biegesteifen Material, das das Aufrollen bei mechanischer Belastung unterbindet.

Weiter vorzugsweise besteht die obere Schicht aus einem harten Polyurethan-Strich, der eine Oberfläche mit einer besonders niedrigen Haft- und Gleitreibung erzeugt, die sich als vorteilhaft für die Anwendung im Umfelde von Druckstellen und/oder Blasen herausgestellt hat.

Die Klebeschicht ist mit der bevorzugten Dicke von 35 bis 50 μm als dünn anzusehen. In einer weiteren vorteilhaften Ausführungsform besteht diese aus einer hautfreundlichen Haftklebemasse aus Polyacrylat, in die zur Erhöhung des Haftvermögens auf Haut ein Tackifier (zum Beispiel ein Kohlenwasserstoff-Harz) eingearbeitet wird.

Die wasserdampfdurchlässige äußere Haftklebeschicht speichert keine Feuchtigkeit und schützt dabei die zentrale flüssigkeitsaufsaugende Klebeschicht vor dem Eindringen von Wasser vom äußeren Rand her (Abdichtung) und verhindert durch sehr dünne Ausführung im Randbereich mögliches Aufrollen (zum Beispiel im Schuh).

Die Polyurethan-Matrix ist insbesondere transparent, stark wasserdampfdurchlässig und klebend. Zur Speicherung von Flüssigkeit wird vorzugsweise ein Superabsorber-Polymer auf Polyacrylatbasis als Pulver eingearbeitet.

Die Polyurethan-Matrix dient als gute Polsterung (konvexe Form, maximale Dicke bevorzugt ca. 1,6 mm) und als Speicher von Exudat, das aus der Druckstelle und/oder Blase austritt.

Geeignete Polyurethane sind Gegenstand der DE 196 18 825, in der hydrophile, selbstklebende Polyurethan-Gele offenbart werden, die bestehen aus

a) 2 bis 6 Hydroxylgruppen aufweisenden Polyetherpolyolen mit OH-Zahlen von 20 bis 112 und einem Ethylenoxid (EO)-Gehalt von ≥ 10 Gew.-%,
b) Antioxidantien,
c) in den Polyolen a) löslichen Wismut-(HI)-Carboxylaten auf Basis von Carbonsäuren mit 2 bis 18 C-Atomen als Katalysatoren sowie
d) Hexamethylendiisocyanat,

mit einem Produkt der Funktionalitäten der Polyurethan-bildenden Komponenten a) und d) von mindestens 5,2, wobei die Katalysatormenge c) 0,005 bis 0,25 Gew.-%, bezogen auf das Polyol a) beträgt, die Menge an Antioxidantien b) im Bereich von 0,1 bis 1,0 Gew.-%, bezogen auf Polyol a) liegt und ein Verhältnis von freien NCO-Gruppen der Komponente d) zu den freien OH-Gruppen der Komponente a) (Isocyanatkennzahl) im Bereich von 0,30 bis 0,70 gewählt wird.

Es werden bevorzugt 3 bis 4, ganz besonders bevorzugt 4-Hydroxylgruppen aufweisende Polyetherpolyole eingesetzt mit einer OH-Zahl im Bereich von 20 bis 112, bevorzugt 30 bis 56. Der Ethylenoxidgehalt liegt bei den erfindungsgemäß eingesetzten Polyetherpolyolen bei vorzugsweise ≥20 Gew.-%.

Die Polyetherpolyole sind als solche an sich bekannt und werden zum Beispiel durch Polymerisation von Epoxiden, wie Ethylenoxid, Propylenoxid, Butylenoxid oder Tetrahydrofuran, mit sich selbst oder durch Anlagerung dieser Epoxide, vorzugsweise von Ethylenoxid und Propylenoxid - gegebenenfalls im Gemisch untereinander oder separat nacheinander - an Starterkomponenten mit mindestens zwei reaktionsfähigen Wasserstoffatomen, wie Wasser, Ethylenglykol, Propylenglykol, Diethylenglykol, Dipropylenglykol, Glyzerin, Trimethylolpropan, Pentaerythrit, Sorbit oder Succrose, hergestellt. Vertreter der genannten, zu verwendenden höhermolekularen Polyhydroxylverbindungen sind zum Beispiel in High Polymers, Vol. XVI, "Polyurethanes, Chemistry and Technology" (Saunders-Frisch, Interscience Publishers, New York, Bd 1, 1962, S. 32-42) aufgeführt.

Als Isocyanatkomponente wird monomeres oder trimerisiertes Hexamethylendiisocyanat oder durch Biuret-, Uretdion-, Allophanatgruppen oder durch Prepolymerisierung mit Polyetherpolyolen oder Mischungen von Polyetherpolyolen auf Basis der bekannten Starterkomponenten mit 2 oder > 2 reaktionsfähigen H-Atomen und Epoxiden, wie Ethylenoxid oder Propylenoxid einer OH-Zahl von ≤ 850, bevorzugt 100 bis 600, modifiziertes Hexamethylendiisocyanat eingesetzt. Bevorzugt ist der Einsatz von modifiziertem Hexamethylendiisocyanat, insbesondere durch Prepolymerisierung mit Polyetherdiolen der OH-Zahl 200 bis 600 modifiziertes Hexamethylendiisocyanat. Ganz besonders bevorzugt sind Modifizierungen des Hexamethylendiisocyanats mit Polyetherdiolen der OH-Zahl 200-600, deren Restgehalt an monomeren Hexamethylendiisocyanat unter 0,5 Gew.-% liegt.

Als Katalysatoren kommen für die erfindungsgemäßen Polyurethangele in den wasserfreien Polyetherpolyolen a) lösliche Wismut(III)-Carboxylate auf Basis linearer, verzweigter, gesättigter oder ungesättigter Carbonsäuren mit 2 bis 18, vorzugsweise 6 bis 18 C-Atomen in Frage. Bevorzugt sind Bi(III)Salze verzweigter gesättigter Carbonsäuren mit tertiären Carboxylgruppen, wie der 2,2-Dimethyl- Octansäure (zum Beispiel Versatic-Säuren, Shell). Gut geeignet sind Zubereitungen dieser Bi (III) Salze in überschüssigen Anteilen dieser Carbonsäuren. Hervorragend bewährt hat sich eine Lösung von 1 mol des Bi(III)Salzes der Versatic 10-Säure (2,2-Dimethyloctansäure) in einem Überschuß von 3 mol dieser Säure mit einem Bi-Gehalt von ca. 17%.

Es werden die Katalysatoren bevorzugt in Mengen von 0,03 bis 0,1 Gew.-%, bezogen auf das Polyol a), eingesetzt.

Als Antioxidantien kommen für die erfindungsgemäßen Polyurethan-Gele insbesondere sterisch gehinderte phenolische Stabilisatoren, wie BHT (2,6-Di-tert.butyl-4-methylphenol), Vulkanox BKF (2,2 min -Methylen-bis-(6-tert.-butyl-4-methyl phenol) (Bayer AG), Irganox 1010 (Pentaerythrityl-tetrakis-[3-(3,5-ditert.-butyl-4- hydroxyphenyl)-propionat]), Irganox 1076 (Octadecyl-3-(3,5-ditert.-butyl-4- hydroxyphenyl)-propionat) (Ciba-Geigy) oder Tocopherol (Vitamin E) in Betracht. Bevorzugt werden solche vom Typ des $\alpha$-Tocopherol eingesetzt.

Die Antioxidantien werden bevorzugt in Mengen von 0,15 bis 0,5 Gew.-%, bezogen auf das Polyol a), eingesetzt. Die Isocyanatkennzahl (Verhältnis der bei der Reaktion eingesetzten freien NCO-Gruppen zu den freien OH-Gruppen) der erfindungsgemäßen Polyurethan-Gelmassen liegt je nach der Funktionalität der eingesetzten Isocyanat- und Polyolkomponenten im Bereich von 0,30 bis 0,70, bevorzugt im Bereich von 0,45 bis 0,60. Die für eine Gelbildung erforderliche Isocyanatkennzahl kann sehr einfach nach der folgenden Formel abgeschätzt werden:

$$ f_{(Polyol)} \bullet (f_{(Isocyanat)} - 1) \bullet Kennzahl \approx 2 $$

$$ Kennzahl \approx \frac{2}{f_{(Polyol)} \bullet (f_{(Isocyanat)} - 1)} $$

f: Funktionalität der isocyanat- oder Polyolkomponente

Je nach angestrebter Klebrigkeit oder Elastizität des Gels kann die tatsächlich zu verwendende isocyanatkennzahl um bis zu + 20% von dem berechneten Wert abweichen.

Die erfindungsgemäßen Polyurethan-Gelmassen werden hergestellt nach üblichen Verfahren, wie sie beispielsweise beschrieben sind in Becker/Braun, Kunststoff- Handbuch, Bd. 7, Polyurethane, S. 121 ff, Carl-Hauser, 1983.

Weiter vorzugsweise kommen Polyurethane zum Einsatz, wie sie in der EP 0 665 856 B1 offenbart sind. Die hydrophilen Polyurethangelschäume sind demnach erhältlich aus

1. einem Polyurethangel, welches

(A) 25-62 Gew.-%, vorzugsweise 30-60 Gew.-%, besonders bevorzugt 40-57 Gew.-%, bezogen auf die Summe aus (A) und (B), eines kovalent vemetzten Polyurethans als hochmolekulare Matrix und

(B) 75-38 Gew.-%, vorzugsweise 70-40 Gew.-%, besonders bevorzugt 60-43 Gew.-%, bezogen auf die Summe aus (A) und (B) einer oder mehrerer in der Matrix durch Nebenvalenzkräfte fest gebundenen Polyhydroxylverbindungen mit einem mittleren Molekulargewicht zwischen 1000 und 12000, vorzugsweise zwischen 1500 und 8000, besonders bevorzugt zwischen 2000 und 6000, und einer mittleren OH-Zahl zwischen 20 und 112, vorzugsweise zwischen 25 und 84, besonders bevorzugt zwischen 28 und 56, als flüssigem Dispersionsmittel, wobei das Dispersionsmittel im wesentlichen frei ist an Hydroxylverbindungen mit einem Molekulargewicht unter 800, vorzugsweise unter 1000, besonders bevorzugt unter 1500, sowie gegebenenfalls

(C) 0 bis 100 Gew.-%, bezogen auf die Summe aus (A) und (B), an Füll- und/oder Zusatzstoffen enthält,

und welches erhältlich ist durch Umsetzung einer Mischung von

a) einem oder mehreren Polyisocyanaten,

b) einer oder mehreren Polyhydroxylverbindungen mit einem mittleren Molekulargewicht zwischen 1000 und 12000, und einer mittleren OH-Zahl zwischen 20 und 112,

c) gegebenenfalls Katalysatoren oder Beschleunigem für die Reaktion zwischen Isocyanat- und Hydroxylgruppen sowie gegebenenfalls

d) aus der Polyurethanchemie an sich bekannten Füll- und Zusatzstoffen,

wobei diese Mischung im wesentlichen frei ist von Hydroxylverbindungen mit einem Molekulargewicht unter 800, die mittlere Funktionalität der Polyisocyanate ($F_I$) zwischen 2 und 4 liegt, die mittlere Funktionalität der Polyhydroxylverbindung ($F_p$) zwischen 3 und 6 beträgt und die Isocyanatkennzahl (K) der Formel

$$K = \frac{300 \pm X}{(F_I \bullet F_p) - 1} + 7$$

gehorcht, in welcher $X \leq 120$, vorzugsweise $X \leq 100$, besonders bevorzugt $X \leq 90$ ist und die Kennzahl K bei Werten zwischen 15 und 70 liegt, wobei die angegebenen Mittelwerte von Molekulargewicht und OH-Zahl als Zahlenmittel zu verstehen sind,

2. einem Wasser absorbierenden Material und

3. einem nichtwäßrigen Schäumungsmittel.

Die Polyurethangele können aus den an sich aus der Polyurethanchemie bekannten Ausgangsverbindungen nach an sich bekannten Verfahren hergestellt werden, wie sie zum Beispiel in DE 31 03 499 A1, DE 31 03 500 A1 und EP 0 147 588 A1 beschrieben werden. Wesentlich ist jedoch, daß bei der Auswahl der gelbbildenden Komponenten die oben definierten Bedingungen eingehalten werden, da sonst anstelle von selbsthaftenden Gelen klebfreie, elastische Gele erhalten werden.

Bevorzugte Polyhydroxylverbindungen sind Polyetherpolyole, wie sie in den oben genannten Offenlegungsschriften ausführlich genannt sind.

Als Polyisocyanatkomponenten sind sowohl (cyclo)aliphatische als auch aromatische Isocyanate geeignet. Bevorzugte (cyclo)aliphatische Polyisocyanate sind 1,6-Hexamethylen-diisocyanat sowie dessen Biurete und Trimerisate bzw. hydrierte Diphenylmethandiisocyanat ("MDI")-Typen. Bevorzugte aromatischen Polyisocyanate sind solche, die durch Destillation erhalten werden, wie MDI-Gemische aus 4,4'- und 2,4'-Isomeren oder 4,4'-MDI, sowie Toluylendiisocyanat ("TDI")-Typen.

Die Diisocyanate können insbesondere zum Beispiel aus der Gruppe der unmodifizierten aromatischen oder aliphatischen Diisocyanate oder aber aus durch Prepolymerisierung mit Aminen, Polyolen oder Polyetherpolyolen gebildeten modifizierten Produkten gewählt werden.

Die Polyurethan-Gele können gegebenenfalls aus der Polyurethan-Chemie an sich bekannte Zusatzstoffe enthalten, wie zum Beispiel Füllstoffe und Kurzfasem auf anorganischer oder organischer Basis, Metallpigmente, oberflächenaktive Substanzen oder flüssige Streckmittel wie Substanzen mit einem Siedepunkt von über 150 °C.

Als organische Füllstoffe seien beispielsweise Schwerspat, Kreide, Gips, Kieserit, Soda, Titandioxid, Ceroxid, Quarzsand, Kaolin, Russ und Mikrohohlkugeln genannt.

An organischen Füllstoffen können zum Beispiel Pulver auf Basis von Polystyrol, Polyvinylchlorid, Hamstoff-Formaldehyd und Polyhydrazodicarbonamid eingesetzt werden. Als Kurzfasem kommen zum Beispiel Glasfasem von 0,1 - 1 mm Länge oder Fasem organischer Herkunft, wie zum Beispiel Polyester- oder Polyamidfasern, in Frage. Metallpulver, wie zum Beispiel Eisen oder Kupferpulver, können ebenfalls bei der Gelbildung mitverwendet werden. Um den Gelen die gewünschte Färbung zu verleihen, können die bei der Einfärbung von Polyurethanen an sich bekannten Farbstoffe oder Farbpigmente auf organischer oder anorganischer Basis verwendet werden, wie zum Beispiel Eisenoxid- oder Chromoxidpigmente, Pigmente auf Phthalocyanin- oder Monoazo-Basis. Als oberflächenaktive Substanzen seien zum Beispiel Cellulosepulver, Aktivkohle und Kieselsäurepräparate genannt.

Zur Modifizierung der Hafteigenschaften der Gele können gegebenenfalls Zusätze von polymeren Vinylverbindungen, Polyacrylaten und sonstigen in der Klebstoff-Technik üblichen Copolymeren bzw. auch Klebemittel auf Naturstoffbasis bis zu einem Gehalt von 10 Gew.-%, bezogen auf das Gewicht der Gelmasse, zugegeben werden.

Bevorzugte Wasser absorbierende Materialien sind als Superabsorber bekannte Wasser absorbierende Salze von Polyacrylaten und deren Copolymeren, insbesondere die Natrium- oder Kaliumsalze. Sie können unvernetzt oder vernetzt sein und sind auch als Handelsprodukte erhältlich. Insbesondere sind solche Produkte geeignet, wie sie in der DE 37 13 601 A1 offenbart werden und auch Superabsorber der neuen Generation mit nur noch geringen Anteilen an austrockenbarem Wasser und hohem Quellvermögen unter Druck.

Bevorzugte Produkte sind schwach vemetzte Polymerisate auf der Basis Acrylsäure/Natriumacrylat Solche Natriumpolyacrylate sind als Favor 22-SK (Chemische Fabrik Stockhausen GmbH, Deutschland) erhältlich.

Weitere Absorber, zum Beispiel Carboxymethylcellulose und Karaya, sind ebenfalls geeignet.

Der Schäumungsgrad läßt sich durch die eingearbeiteten Mengen an Schäumungsmittel in weiten Grenzen variieren.

Gegebenenfalls wird das selbstklebende Produkt durch ein silikonisiertes Papier oder eine insbesondere silikonisierte Folie abgedeckt, so daß die klebende Seite während der Lagerung geschützt ist.

Der erfindungsgemäße transparente Verband zeigt langanhaltendes Haftvermögen an stark konturierten Homhautflächen des menschlichen Körpers, das durch den speziellen Produktaufbau erreicht wird. Der Verband zeigt gute polsternde Eigenschaft ohne Beeinträchtigung des Tragekomforts im Schuh durch spezielle konvexe Pflasterform. Das Aufrollen im Schuh des Pflasters durch mechanische Beanspruchung wird durch ein ausreichend dickes und biegesteifes Trägermaterial verhindert.

Eine Absorption von Exudat aus Druckstellen wird durch eine Superabsorber enthaltende klebende Polyurethan-Matrix erreicht.

Es findet ein ausreichender Feuchtigkeitstransport von der Hornhaut durch das Pflaster nach außen durch Kombination geeigneter Komponenten von Träger und Klebemassen bei gleichzeitiger Generierung eines feuchten Wundheilungsmilieus statt.

Der neue Verband zeichnet sich durch signifikant verlängerte Tragedauer, sehr gute Komforteigenschaften, rückstandsfreie Entfembarkeit und optische Unauffälligkeit gegenüber herkömmlichen Pflastern aus, zeigt also eine optimale Haftung an Hornhautpartien (Ferse oder Handballen) aufgrund der zentralen, stark polsternden, flüssigkeitsabsorbierenden, schwach klebenden Haftklebezone und der stark klebenden, nichtabsorbierenden äußeren Zone sowie des besonders beschaffenen wasserdampfdurchlässigen Trägermaterials.

Im folgenden sollen anhand zweier Bilder sowie mehrerer Beispiele besonders vorteilhafte Ausführungsformen des Verbands dargestellt werden, ohne damit die Erfindung unnötig einschränken zu wollen.

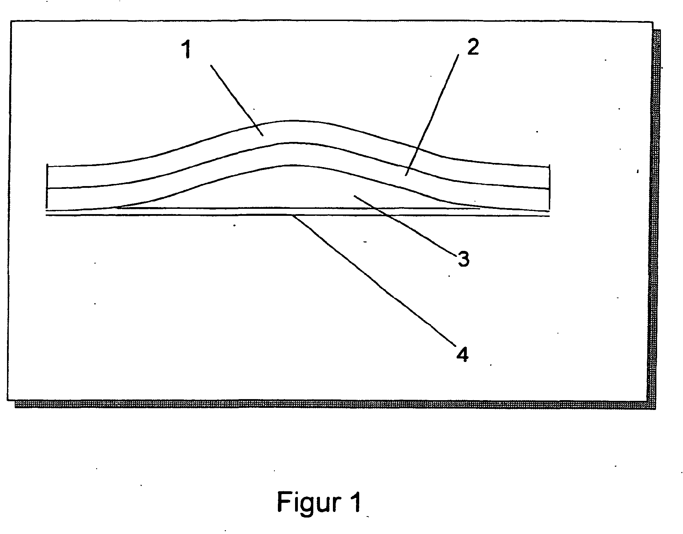

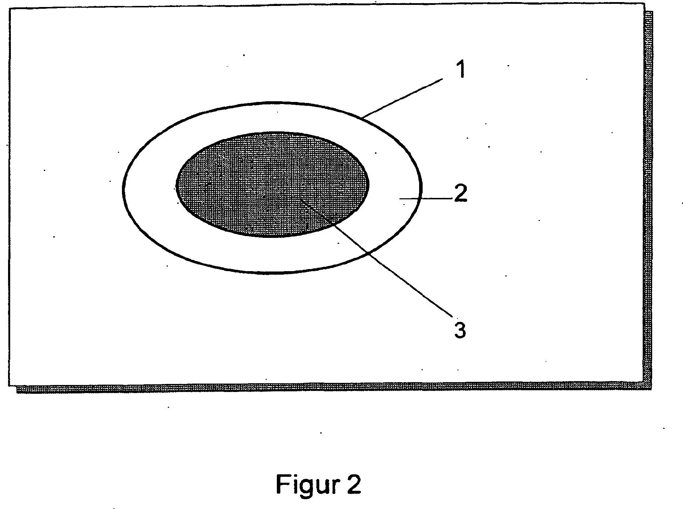

Die Figur 1 und die Figur 2 verdeutlichen die bevorzugte geometrische Form des Verbands, wie er insbesondere für Blasenpflaster zum Einsatz kommt.

Das Pflaster weist eine ellipsoide Form auf, besteht aus einer Trägerfolie 1, die mit einer Klebeschicht 2 vollflächig beschichtet ist.

Mittig auf der Klebeschicht 2 ist eine wasserdampfdurchlässige Polyurethan-Matrix 3 vorhanden, die sich zum Rand der Klebeschicht 2 hin abschrägt. In der hier gezeigten Ausführungsform des Blasenpflasters ist die gesamte Peripherie der Klebeschicht 2 nicht mit der Polyurethan-Matrix 3 bedeckt. Es ergeben sich auf diese Weise zwei konzentrische Zonen chemisch unterschiedlicher Klebemassen 2, 3, welche sich hinsichtlich Haftvermögen, Absorptionsvermögen und Polstereigenschaft unterscheiden.

Die Polyurethan-Matrix 3 ist im wesentlichen halbkonvex ausgeformt, ist demgemäß einer halbkonvexen Linse vergleichbar.

Schließlich ist das Pflaster mit einem silikonisierten Papier 4 eingedeckt, um eine Verschmutzung oder Kontamination der Klebmasse 2 oder der Matrix 3 zu vermeiden.

### Beispiele

### Beispiel 1: Verband insbesondere zur Versorgung von Druckstellen und Blasen

a) Trägermaterial:

Das Trägermaterial des Pflasters besteht aus einer mehrschichtigen 50 $\mu$m dicken Folie eines Polyesterurethans (Impranil-Type, Bayer AG, leverkusen).

Der Schichtaufbau wird durch wiederholtes Gießen einer ca. 20g/m$^2$ Polyesterurethan-Schicht auf einer ca. 100 $\mu$m dicken PE-Folie (Waloplast, Wolf Walsrode) hergestellt.

Bei diesem Prozeß wird die Zusammensetzung des Polyetherurethans in der Weise sukzessive geändert, daß der Anteil weicher Polyether-Blöcke bei jedem Strich zunimmt und damit der härteste Strich als äußerer Deckstrich das Pflasters nach außen abschließt. Die PE-Folie wird in einem späteren Verarbeitungsschritt von dem PU-Träger entfernt

Tabelle 1: Physikalische Eigenschaften des PU-Trägematerials:

| Prüfung | Prüfvorschrift | Einheit | Typ A 60 g/m$^2$ | Typ B 80 g/m$^2$ |
|---|---|---|---|---|
| Dicke | DIN 53370 | $\mu$m | 51 | 70 |
| Flächengewicht | DIN 53352 | g/m$^2$ | 60 | 82 |
| Höchstzugkraft | DIN EN ISO 527-1/3 | N/cm | 6,6 | 9,6 |
| Elastische Verformung | Beiersdorf Prüfanweisung AVS00099* | % | 40,0 | 40,8 |
| Biegesteifigkeit cd | DIN 53362 | cN*cm$^2$ | 0,024 | - |
| Wasserdampfdurchlässigkeit | Beiersdorf Prüfanweisung 3VS00008** | g/(m$^2$*24h) | 770 | 687 |

Tabelle fortgesetzt

| Prüfung | Prüfvorschrift | Einheit | Typ A 60 g/m$^2$ | Typ B 80 g/m$^2$ |
|---|---|---|---|---|
| Reibungszahl | DIN 53375 | - | 0,56 | 0,59 |

* Bei der Beiersdorf Prüfanweisung AVS00099* werden aus dem Material quer zur Fertigungsrichtung Proben mit 20 mm Breite und 100 mm Länge geschnitten.

Die Proben werden 24 Std. bei 23 $\pm$ 2 °C und 50 $\pm$ 5 % rel. LF vorkonditioniert. Jede Probe wird in einer Zugprüfmaschine mit einer Prüfgeschwindigkeit von 200 mm/min 5 mal um 50% gedehnt und wieder entspannt (Hysterese). Die Kraft-Dehnungs-Kurve wird aufgenommen, der das Ergebnis nach dem 5. Zyklus zu entnehmen ist.

** Bei der Beiersdorf Prüfanweisung 3VS00008** erfolgt die Prüfung nach ASTM E 96 (water method), mit folgenden Abweichungen: • Die Öffnung des Prüfgefäßes beträgt 804 mm$^2$ • Das Material wird 24 Std. vorkonditioniert bei 23 $\pm$ 2 °C und 50 $\pm$ 5 % rel. LF • Der Abstand zwischen Wasserspiegel im Prüfgefäß und der Probe beträgt 35 $\pm$ 5 mm • Das Rückwiegen der mit Proben bestückten Prüfgefäße erfolgt nach 24 Std., in denen diese im Klimaschrank bei 37 $\pm$ 1,5 °C und 30 $\pm$ 3 % rel. LF gelagert werden.

b) Haftklebebeschichtung äußere Zone

Die Acrylat-Haftklebemasse wird auf ein silikonisiertes Papier gestrichen und abgerakelt. Die vollflächige Beschichtung wird dann im Trockenkanal bei Temperaturen von 60 bis 100 °C getrocknet. Der Träger(-verbund) wird nach dem Trockenkanal zukaschiert. Das Verbundprodukt wird ca. 1 s lang mit einer Strahlung von 11 bis 12 kW bei 254 nm bestrahlt, um die Klebemasse zu vernetzen.

Zum Einsatz kommt eine Acrylat-Haftklebemasse Typ C mit einem Flächengewicht von 50 g/m$^2$, bestehend aus einem Copolymerisat aus Ethylhexylacrylat, n-Butylacrylat und Acrylsäure.

Dem Copolymerisat wird ein Klebharz aus der Gruppe der C$_5$-Kohlenwasserstoffharze zugesetzt (zum Beispiel Escorez 5340 von Exxon).

Tabelle 2: Physikalische Daten der Haftklebemasse auf Hostaphan (PET)

| Methode | Einheit | Acrylat-Haftklebemasse Typ C + Escorez 5340 | Vergleichsbeispiel: Acrylat-Haftklebemasse Typ C |
|---|---|---|---|
| Masseauftrag | g/m$^2$ | 50 | 50 |
| Klebkraft Stahl/Peel-force | N/cm | 4,0 | 3,1 |
| Tack (Rolling Ball)* | cm | 5,3 | 6,5 |
| * gemessen nach PSTC-6 ("Pressure Sensitive Tape Council" Testmethode 6) | | | |

Tabelle 3: Physikalische Daten Haftklebemasse-Beschichtung auf PU-Träger

| Methode | Einheit | Typ D Kombination Träger-Haftklebemasse: Acrylat-Haftklebemasse Typ C + Escorez 5340 auf 60g/m$^2$ Träger (Typ A) | Typ E Kombination Träger-Haftklebemasse: Acrylat-Haftklebemasse Typ C + Escorez 5340 auf 80 g/m$^2$ Träger (Typ B) |
|---|---|---|---|
| Masseauftrag | g/m$^2$ | 56 | 56 |
| Klebkraft Stahl/(Peel-force) | N/cm | 6,7 | 7,1 |
| Wasserdampfdurchlässigkeit* | g/(m$^2$*24h) | 273 | 254 |
| ** Bei der Beiersdorf Prüfanweisung 3VS00008** erfolgt die Prüfung nach ASTM E 96 (water method), mit folgenden Abweichungen: • Die Öffnung des Prüfgefäßes beträgt 804 mm$^2$ • Das Material wird 24 Std. vorkonditioniert bei 23 $\pm$ 2 °C und 50 $\pm$ 5 % rel. LF • Der Abstand zwischen Wasserspiegel im Prüfgefäß und der Probe beträgt 35 $\pm$ 5 mm • Das Rückwiegen der mit Proben bestückten Prüfgefäße erfolgt nach 24 Std., in denen diese im Klimaschrank bei 37 $\pm$ 1,5 °C und 30 $\pm$ 3 % rel. LF gelagert werden. | | | |

c) Haftklebebeschichtung innere Zone (Absorber-Zone)

Der beschichtete Träger wird in eine Gießform eingelegt, die eine konkave, ovale Vertiefung aufweist. Die Vertiefung ist im Zentrum des Ovals ca. 1,6 mm tief und verringert sich sukzessive zu den Rändern.

Der beschichtete Träger wird mit der Haftklebebeschichtung zur Oberseite hin in die Gießform eingelegt und 0,7 g der reaktiven Polyurethanmasse (Levagel, Diisocyanat, Katalysator, Superabsorber, Kennzahl 45 bis 55) in der Mitte der Kavität aufgetragen. Der Verbund wird mit einem silikonisierten, PE beschichteten Papier abgedeckt und mit einem Gewicht bis zur endgültigen Aushärtung belastet.

Nach Aushärtung wird der PE-Film entfernt und konzentrisch um die zentrale Absorber-Zone der äußere Pflasterrand oval ausgestanzt.

Tabelle 4: Physikalische Daten des Pflasters im Bereich der Absorberzone

| Methode | Prüfvorschrift | Einheit | Verband gemäß Bsp.1 auf Basis des Typs D |
|---|---|---|---|
| Flüssigkeitsaufnahme | Beiersdorf Prüfanweisung 3VS00004* | g/(g* 3h) | ≥ 1,0 |
| Wasserdampfdurchlässigkeit | Beiersdorf Prüfanweisung 3VS00008** | g/(m$^2$*24h) | > 200 |

*: Beiersdorf Prüfanweisung 3VS00004* wird je Pflaster mittig eine Probe mit einem Durchmesser von 15 mm ausgestanzt und eine Stunde bei 23 ± 2 °C und 50 ± 5 % rel. LF vorkonditioniert. Die Proben werden gewogen und für 3 Stunden vollständig in physiologische 23 ± 0,5 °C warme Natriumchloridlösung getaucht. Die Proben werden erneut gewogen und die Flüssigkeitsaufnahme berechnet.

** Bei der Beiersdorf Prüfanweisung 3VS00008** erfolgt die Prüfung nach ASTM E 96 (water method), mit folgenden Abweichungen: • Die Öffnung des Prüfgefäßes beträgt 804 mm$^2$ • Das Material wird 24 Std. vorkonditioniert bei 23 ± 2 °C und 50 ± 5 %rel. LF • Der Abstand zwischen Wasserspiegel im Prüfgefäß und der Probe beträgt 35 ± 5 mm • Das Rückwiegen der mit Proben bestückten Prüfgefäße erfolgt nach 24 Std., in denen diese im Klimaschrank bei 37 ± 1,5 °C und 30 ± 3 % rel. LF gelagert werden.

Im Ergebnis erhält man ein selbstklebendes Wundversorgungsprodukt mit einer äußeren stark klebenden Zone der Abmessung 43 x 68 mm und einer inneren, polstemden und flüssigkeitsabsorbierenden Zone mit der Abmessung 25 x 46 mm, die ein feuchtes Wundheilungsmilieu generiert.

Der wasserdampfdurchlässige Polyurethan-Träger bietet eine Barriere gegen Wasser und Bakterien und verhindert Mazeration der Haut unter dem Pflaster.

**Beispiel 2: Verband insbesondere zur Abdeckung von Wunden**

a) Trägermaterial

Das Trägermaterial des Pflasters besteht aus einer 35 μm dicken Folie eines Polyesterurethans (Impranil-Type, Bayer AG, Leverkusen). Diese wird durch wiederholtes Gießen einer ca. 20g/m$^2$ Polyesterurethan-Schicht auf einer ca. 100 μm dicken PE-Folie (Waloplast, Wolf Walsrode) hergestellt, die bei der Applikation des Pflasters entfernt wird.

Tabelle 5: Physikalische Eigenschaften des PU-Trägematerials:

| Prüfung | Prüfvorschrift | Einheit | Typ F 40 g/m$^2$ |
|---|---|---|---|
| Dicke | DIN 53370 | μm | 34 |
| Flächengewicht | DIN 53352 | g/m$^2$ | 39 |
| Höchstzugkraft | DIN EN ISO 527-1/3 | N/cm | > 8 |
| Elastische Verformung | Beiersdorf Prüfanweisung AVS00099* | % | > 42 |

Tabelle fortgesetzt

| Prüfung | Prüfvorschrift | Einheit | Typ F 40 g/m$^2$ |
|---|---|---|---|
| Wasserdampfdurchlässigkeit | Beiersdorf Prüfanweisung 3VS00008** | g/(m$^2$*24h) | > 1300 |
| *: Beiersdorf Prüfanweisung 3VS00004* wird je Pflaster mittig eine Probe mit einem Durchmesser von 15 mm ausgestanzt und eine Stunde bei 23 ± 2 °C und 50 ± 5 % rel. LF vorkonditioniert. Die Proben werden gewogen und für 3 Stunden vollständig in physiologische 23 ± 0,5 °C warme Natriumchloridlösung getaucht. Die Proben werden erneut gewogen und die Flüssigkeitsaufnahme berechnet.<br>** Bei der Beiersdorf Prüfanweisung 3VS00008** erfolgt die Prüfung nach ASTM E 96 (water method), mit folgenden Abweichungen: • Die Öffnung des Prüfgefäßes beträgt 804 mm$^2$ • Das Material wird 24 Std. vorkonditioniert bei 23 ± 2 °C und 50 ± 5 % rel. LF • Der Abstand zwischen Wasserspiegel im Prüfgefäß und der Probe beträgt 35 ± 5 mm • Das Rückwiegen der mit Proben bestückten Prüfgefäße erfolgt nach 24 Std., in denen diese im Klimaschrank bei 37 ± 1,5 °C und 30 ± 3 % rel. LF gelagert werden. | | | |

b) Haftklebebeschichtung äußere Zone

Die Acrylat-Haftklebemasse wird auf ein silikonisiertes Papier gestrichen und abgerakelt. Die vollflächige Beschichtung wird dann im Trockenkanal bei Temperaturen von 60 bis 100 °C getrocknet. Der Träger(-verbund) wird nach dem Trockenkanal zukaschiert. Das Verbundprodukt wird ca. 1 s lang mit einer Strahlung von 11 bis 12 kW bei 254 nm bestrahlt, um die Klebemasse zu vernetzen.

Zum Einsatz kommt eine Acrylat-Haftklebemasse Typ C mit einem Flächengewicht von 50 g/m$^2$, bestehend aus einem Copolymerisat aus Ethylhexylacrylat, n-Butylacrylat und Acrylsäure. Die physikalischen Daten der Haftklebemasse sind Tabelle 2 unter Punkt b) in Beispiel 1 zu entnehmen.

Tabelle 6: Physikalische Daten Haftklebemasse-Beschichtung auf PU-Träger

| Methode | Einheit | Typ G. Kombination Träger-Haftklebemasse: Acrylat-Haftklebemasse Typ C auf 40g/m$^2$ Träger (Typ F) |
|---|---|---|
| Masseauftrag | g/m$^2$ | 38 |
| Klebkraft Stahl/(Peel-force) | N/cm | 3,0 |
| Wasserdampfdurchlässigkeit* | g/(m$^2$*24h) | > 300 |
| * Bei der Beiersdorf Prüfanweisung 3VS00008* erfolgt die Prüfung nach ASTM E 96 (water method), mit folgenden Abweichungen: • Die Öffnung des Prüfgefäßes beträgt 804 mm$^2$ • Das Material wird 24 Std. vorkonditioniert bei 23 ± 2 °C und 50 ± 5 % rel. LF • Der Abstand zwischen Wasserspiegel im Prüfgefäß und der Probe beträgt 35 ± 5 mm • Das Rückwiegen der mit Proben bestückten Prüfgefäße erfolgt nach 24 Std., in denen diese im Klimaschrank bei 37 t 1,5 °C und 30 ± 3 % rel. LF gelagert werden. | | |

c) Haftklebebeschichtung innere Zone (Absorber-Zone)

Der beschichtete Träger wird in eine Gießform eingelegt, die eine konkave, ovale Vertiefung aufweist. Die Vertiefung ist im Zentrum des Ovals ca. 1,3 mm tief und verringert sich sukzessive zu den Rändern.

Der beschichtete Träger wird mit der Haftklebebeschichtung zur Oberseite hin in die Gießform eingelegt und 1,6 g der reaktiven Polyurethanmasse (Levagel, Diisocyanat, Katalysator, Superabsorber, Titandioxid; Kennzahl 45 bis 55) in der Mitte der Kavität aufgetragen. Der Verbund wird mit einem silikonisierten, PE beschichteten Papier abgedeckt und mit einem Gewicht bis zur endgültigen Aushärtung belastet.

Nach Aushärtung wird der PE-Film mit einer Abziehhilfe versehen und konzentrisch um die zentrale Absorber-Zone der äußere Pflasterrand oval ausgestanzt. An der Abziehhilfe wird der PE-Film von dem PU-Träger entfernt, nachdem das Pflaster auf die Wunde geklebt wurde.

Tabelle 7: Physikalische Daten des Pflasters im Bereich der Absorberzone

| Methode | Prüfvorschrift | Einheit | Verband gemäß Bsp.2 auf Basis des Typs G |
|---|---|---|---|
| Flüssigkeitsaufnahme | Beiersdorf Prüfanweisung 3VS00004* | g/(g *3h) | ≥ 1,0 |

Tabelle fortgesetzt

| Methode | Prüfvorschrift | Einheit | Verband gemäß Bsp.2 auf Basis des Typs G |
|---|---|---|---|
| Wasserdampfdurchlässigkeit | Beiersdorf Prüfanweisung 3VS00008** | g/(m$^2$*24h) | > 250 |

* Beiersdorf Prüfanweisung 3VS00004* wird je Pflaster mittig eine Probe mit einem Durchmesser von 15 mm ausgestanzt und eine Stunde bei 23 ± 2°C und 50 ± 5 % rel. LF vorkonditioniert. Die Proben werden gewogen und für 3 Stunden vollständig in physiologische 23 ± 0,5 °C warme Natriumchloridlösung getaucht. Die Proben werden erneut gewogen und die Flüssigkeitsaufnahme berechnet.

** Bei der Beiersdorf Prüfanweisung 3VS00008** erfolgt die Prüfung nach ASTM E 96 (water method), mit folgenden Abweichungen:

• Die Öffnung des Prüfgefäßes beträgt 804 mm$^2$ • Das Material wird 24 Std. vorkonditioniert bei 23 ± 2 °C und 50 ± 5 % rel. LF • Der Abstand zwischen Wasserspiegel im Prüfgefäß und der Probe beträgt 35 ± 5 mm • Das Rückwiegen der mit Proben bestückten Prüfgefäße erfolgt nach 24 Std., in denen diese im Klimaschrank bei 37 ± 1,5 °C und 30 ± 3 % rel. LF gelagert werden.

Im Ergebnis erhält man ein selbstklebendes Wundversorgungsprodukt mit einer äußeren stark klebenden Zone der Abmessung 66 x 110 mm und einer inneren, polsternden und flüssigkeitsabsorbierenden Zone mit der Abmessung 40 x 80 mm, die ein feuchtes Wundheilungsmilieu generiert. Durch das Titandioxid erscheint die Absorptionszone milchig und deckt somit die Wunde nicht nur physisch sondern auch optisch ab.

Der wasserdampfdurchlässige Polyurethan-Träger bietet eine Barriere gegen Wasser und Bakterien und verhindert Mazeration der Haut unter dem Pflaster.

**Patentansprüche**

1. Verband insbesondere zur Abdeckung von Wunden und Verhütung oder Behandlung von Blasen, umfassend eine wasserdampfdurchlässige Trägerfolie, auf die vollflächig eine Klebeschicht aufgetragen ist, wobei mittig auf der Klebeschicht eine wasserdampfdurchlässige selbstklebende Polyurethan-Matrix vorhanden ist, die sich von einem Punkt, insbesondere mittig gelegen, zum Rand der Klebeschicht hin abschrägt, wobei die Peripherie der Klebeschicht zumindest teilweise nicht von der Polyurethan-Matrix bedeckt ist.

2. Verband nach Anspruch 1, **dadurch gekennzeichnet, dass** die Trägerfolie aus Polyurethan besteht.

3. Verband nach den Ansprüchen 1 oder 2, **dadurch gekennzeichnet, dass** die Trägerfolie eine Dicke zwischen 60 bis 80 μm aufweist.

4. Verband nach einem der Ansprüche 1 bis 3, **dadurch gekennzeichnet, dass** die Klebeschicht aus Polyacrylat besteht.

5. Verband nach einen der Ansprüche 1 bis 4, **dadurch gekennzeichnet, dass** die Klebeschicht eine Dicke zwischen 35 bis 50 μm aufweist.

6. Verband nach einem der vorherigen Ansprüche, **dadurch gekennzeichnet, dass** der Verband über seine ganze Breite bis zum Gebrauch mit einem klebstoffabweisenden Trägermaterial, wie silikonisiertes Papier, abgedeckt ist.

**Revendications**

1. Pansement, en particulier pour recouvrir des plaies et prévenir ou traiter les cloches, comprenant une feuille support perméable à la vapeur d'eau, sur laquelle est appliquée, sur toute la surface, une couche adhésive, une matrice auto-adhésive de polyuréthane, perméable à la vapeur d'eau, se trouvant au centre de la couche adhésive, qui part en biais à partir d'un point, en particulier au centre, vers le bord de la couche adhésive, la périphérie de la couche adhésive n'étant pas revêtue par la matrice de polyuréthane, du moins partiellement.

2. Pansement selon la revendication 1, **caractérisé en ce que** la feuille support est en polyuréthane.

**3.** Pansement selon les revendications 1 ou 2, **caractérisé en ce que** la feuille support présente une épaisseur entre 60 et 80 μm.

**4.** Pansement selon l'une quelconque des revendications 1 à 3, **caractérisé en ce que** la couche adhésive est en polyacrylate.

**5.** Pansement selon l'une quelconque des revendications 1 à 4, **caractérisé en ce que** la couche adhésive présente une épaisseur entre 35 et 50 μm.

**6.** Pansement selon l'une quelconque des revendications précédentes, **caractérisé en ce que** le pansement est revêtu sur toute sa largeur, jusqu'à l'utilisation, d'un matériau support repoussant les adhésifs, tel qu'un papier siliconé.

**Claims**

**1.** Dressing, in particular for covering wounds and preventing or treating blisters, comprising a water vapour pervious backing film with an adhesive layer applied across its entire surface, a water vapour pervious and self-adhesive polyurethane matrix being disposed centrally on the adhesive layer and sloping down from an especially central point towards the edge of the adhesive layer, at least part of the periphery of the adhesive layer not being covered by the polyurethane matrix.

**2.** Dressing according to Claim 1, **characterized in that** the backing film comprises polyurethane.

**3.** Dressing according to Claims 1 or 2, **characterized in that** the backing film has a thickness of between 60 to 80 μm.

**4.** Dressing according to one of Claims 1 to 3, **characterized in that** the adhesive layer comprises polyacrylate.

**5.** Dressing according to one of Claims 1 to 4, **characterized in that** the adhesive layer has a thickness of between 35 to 50 μm.

**6.** Dressing according to one of the preceding claims, **characterized in that** the dressing is covered across its entire width with an anti-adhesive backing material, such as siliconized paper, until use.

Figur 1

**Figur 2**